# EUROPÄISCHE PATENTANMELDUNG

(11) **EP 3 489 241 A1**
(43) Veröffentlichungstag der Anmeldung: **29.05.2019**
(21) Anmeldenummer: 17203063.7
(22) Anmeldetag: 22.11.2017
(51) Int. Cl.: C07D 493/04

(54) **VERFAHREN ZUR SYNTHESE VON ISOSORBIDMONO- UND DI-(METH)ACRYLAT**

(71) Anmelder: BASF SE, 67056 Ludwigshafen am Rhein (DE)
(72) Erfinder: BLANCHOT, Mathieu, 67056 Ludwigshafen (DE); FLECKENSTEIN, Christoph, 67056 Ludwigshafen (DE); STENGEL, Ulrik, 67056 Ludwigshafen (DE); URTEL, Bolette, 67056 Ludwigshafen (DE); GERLINGER, Wolfgang, 67056 Ludwigshafen (DE); DELAISTIER, Frank, 67056 Ludwigshafen (DE); HOCK, Gunter, 67056 Ludwigshafen (DE); NAIR, Ritesh, 67056 Ludwigshafen (DE); CURATO, Christina, Pearland, TX 77584 (US); HURTADO, Juan C., Houston, TX 77079 (US)
(74) Vertreter: Féaux de Lacroix, Stefan

(57) **Zusammenfassung**

Verfahren zur Herstellung von Isosorbid-Estern der (Meth)acrylsäure oder einem Derivat davon durch Umsetzung von (Meth)acrylsäure oder einem Derivat davon mit Isosorbid oder einem Derivat davon in Gegenwart wenigstens eines die Veresterungsreaktion katalysierenden Enzyms bei einer Reaktionstemperatur von 10 bis 100°C.

## Beschreibung

Die vorliegende Erfindung betrifft ein Verfahren zur Herstellung von Estern von Isosorbid oder eines Derivats von Isosorbid durch Umsetzung von Isosorbid oder eines Derivats davon mit (Meth)acrylsäure oder einem Derivat davon in Gegenwart wenigstens eines die Veresterungsreaktion katalysierenden Enzyms bei einer Reaktionstemperatur von 10 bis 100°C. Des Weiteren betrifft die vorliegende Erfindung einen Ester, herstellbar durch das erfindungsgemäße Verfahren, sowie dessen Verwendung in Kontaktlinsen oder als Vernetzer oder Haftverbesserer für Dispersionen, die vorzugsweise als Klebstoffe, Anstrichmittel oder Textil-, Leder- oder Papierhilfsmittel und in härtbaren Beschichtungen eingesetzt werden.

Im Allgemeinen sind Verfahren zur Herstellung von Isosorbid-Estern der (Meth)acrylsäure oder einem Derivat davon dem Fachmann aus dem Stand der Technik bereits bekannt.

US 2012/0092426 A1 offenbart eine härtbare Tinte enthaltend funktionalisierte Isosorbide. Die Isosorbid-Verbindungen der allgemeinen Formel (I) gemäß dieser Schrift sind die Diester der Acrylsäure an Isosorbid. Die Diester werden erhalten durch Umsetzung von Isosorbid mit Methacrylsäurechlorid in Gegenwart von Triethylamin in Tetrahydrofuran. Eine weitere genannte Methode zur Herstellung der Di-(meth)acrysläure-Isosorbidester umfasst die Umsetzung von Isosorbid mit Acrylsäure in Gegenwart von para-Toluolsulfonsäure in Toluol.

Wulff et al., Makromol. Chem. 188, 731 bis 740 (1987), offenbaren ebenfalls ein Verfahren zur Herstellung des Diesters von Methacrylsäure an Isosorbid. Dazu wird das Methacrylsäurechlorid in Gegenwart von Triethylamin mit Isosorbid umgesetzt.

Die Verfahren zur Herstellung von (Meth)acrylsäureestern von Isosorbid können bezüglich ihrer Raum-Zeit-Ausbeute noch verbessert werden. Des Weiteren können die aus dem Stand der Technik bekannten Verfahren, auch mit Blick auf die notwendige Reaktionszeit, die benötigt wird, um einen möglichst vollständigen Umsatz zu erhalten, verbessert werden. Des Weiteren ist es bei den Verfahren gemäß dem Stand der Technik oft nachteilig, dass eine Mischung des entsprechenden Mono- und Diesters der (Meth)acrylsäure an Isosorbid erhalten wird. Des Weiteren ist die Reinheit der durch die Verfahren des Standes der Technik erhaltenen (Meth)acrylsäure-Ester von Isosorbid noch zu verbessern.

Aufgabe der vorliegenden Erfindung ist es demnach, ein Verfahren zur Herstellung von Estern von Isosorbid oder eines Derivats davon mit (Meth)acrylsäure oder einem Derivat davon bereitzustellen, welches die oben genannten Nachteile nicht aufweist. Insbesondere soll das Verfahren die gewünschten Produkte in hoher Ausbeute und hoher Reinheit zugänglich machen. Insbesondere soll es durch das erfindungsgemäße Verfahren möglich sein, gezielt den entsprechenden Mono- oder Diester zu erhalten.

Diese Aufgaben werden gelöst durch das erfindungsgemäße Verfahren zur Herstellung von Estern von Isosorbid oder eines Derivats von Isosorbid durch Umsetzung von Isosorbid oder eines Derivats davon mit (Meth)acrylsäure oder einem Derivat davon in Gegenwart wenigstens eines die Veresterungsreaktion katalysierenden Enzyms bei einer Reaktionstemperatur von 10 bis 100°C.

Die erfindungsgemäßen Aufgaben werden des Weiteren gelöst durch den erfindungsgemäß herstellbaren Ester sowie dessen Verwendung in Kontaktlinsen oder als Vernetzer oder Haftverbesserer für Dispersionen, die vorzugsweise als Klebstoffe, Anstrichmittel oder Textil-, Leder- oder Papierhilfsmittel und in härtbaren Beschichtungen eingesetzt werden.

Das erfindungsgemäße Verfahren wird im Folgenden detailliert beschrieben:
Durch das erfindungsgemäße Verfahren können Ester von Isosorbid oder eines Derivats davon mit (Meth)acrylsäure oder einem Derivat davon hergestellt werden. Dem Fachmann ist die Gruppe chemischer Verbindungen der Ester bekannt. Dabei wird in einer organischen Carbonsäure das Proton der Carbonsäure-Funktion durch einen Kohlenstoff enthaltenden Rest ersetzt.

Das erfindungsgemäße Verfahren umfasst die Reaktion von Isosorbid oder eines Derivats davon mit (Meth)acrylsäure oder einem Derivat davon.

In dem erfindungsgemäßen Verfahren wird bevorzugt der entsprechende Mono-, der entsprechende Diester von Isosorbid oder eines Derivats davon oder eine Mischung davon gebildet.

In dem erfindungsgemäßen Verfahren können als Edukte im Allgemeinen (Meth)acrylsäure und alle dem Fachmann bekannten Derivate davon eingesetzt werden. Erfindungsgemäß wird der Begriff (Meth)acrylsäure als Sammelbegriff für Acrylsäure und Methacrylsäure verwandt, d.h. der Begriff beschreibt Acrylsäure, Methacrylsäure oder Mischungen davon.

Erfindungsgemäß bevorzugt als Edukte eingesetzte Derivate der (Meth)acrylsäure sind die entsprechenden Ester von Acrylsäure, d.h. Acrylate, oder von Methacrylsäure, d.h. Methacrylate. Erfindungsgemäß werden unter "Derivat der (Meth)acrylsäure" auch entsprechende Verbindungen verstanden, die an der Doppelbindung andere Substituenten als Wasserstoff oder Methyl aufweisen.

In einer besonders bevorzugten Ausführungsform des erfindungsgemäßen Verfahrens entspricht die als Ausgangsverbindung eingesetzte (Meth)acrylsäure oder ein Derivat davon der allgemeinen Formel (I) 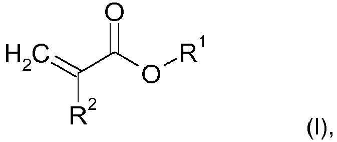 worin R¹ und R² unabhängig voneinander die folgenden Bedeutungen haben:
- R¹: Wasserstoff, Methyl, Ethyl, Propyl, beispielsweise n-Propyl,
- R²: Wasserstoff, aliphatischer, linearer oder verzweigter, gesättigter oder ungesättigter, gegebenenfalls Heteroatome, ausgewählt aus N, O, P, S, und/oder gegebenenfalls wenigstens eine funktionelle Gruppe enthaltender Kohlenstoffrest mit insgesamt 1 bis 24 C-Atomen oder cyclischer, gesättigter oder ungesättigter, gegebenenfalls Heteroatome, ausgewählt aus N, O, P, S, und/oder gegebenenfalls wenigstens eine funktionelle Gruppe enthaltender Kohlenstoffrest mit insgesamt 3 bis 24 C-Atomen oder substituierter oder unsubstituierter, gegebenenfalls Heteroatome, ausgewählt aus N, O, P, S, enthaltender aromatischer Kohlenstoffrest mit insgesamt 5 bis 24 C-Atomen oder funktionelle Gruppen, beispielsweise CN oder SO₃.

Gegebenenfalls an R² vorliegende Substituenten sind beispielsweise Alkylketten mit 1 bis 6 C-Atomen. Gegebenenfalls an R² als Substituenten vorliegende funktionelle Gruppen sind beispielsweise Hydroxy-, Amino-, Keto-, Carbonyl-, Halogenid-, Cyano-, Isocyano- oder SulfatGruppen. In einer bevorzugten Ausführungsform sind die als R² vorliegenden Reste nicht substituiert.

In der allgemeinen Formel (I) ist R¹ bevorzugt ausgewählt aus Wasserstoff oder Methyl.

In der allgemeinen Formel (I) ist R² bevorzugt ausgewählt aus Wasserstoff oder Methyl.

Die vorliegende Erfindung betrifft daher bevorzugt das erfindungsgemäße Verfahren, wobei als Derivat der (Meth)acrylsäure ein Ester, insbesondere ein Methyl-, Ethyl- oder Propylester, eingesetzt wird.

Erfindungsgemäß werden besonders bevorzugt Verbindungen der allgemeinen Formel (I) eingesetzt, in denen R¹ und R² jeweils Wasserstoff (Acrylsäure), R¹ Wasserstoff und R² Methyl (Methacrylsäure), R¹ Methyl und R² Wasserstoff (Acrylsäuremethylester, Methylacrylat) oder R¹ und R² jeweils Methyl (Methacrylsäuremethylester, Methylmethacrylat) bedeuten.

Erfindungsgemäß können auch Mischungen eingesetzt werden, die zwei oder mehr Verbindungen der allgemeinen Formel (I) enthalten.

Verbindungen der allgemeinen Formel (I) bzw. deren Mischungen können nach dem Fachmann bekannten Verfahren hergestellt werden oder sind kommerziell erhältlich.

Bevorzugt entspricht das Isosorbid oder das Derivat davon der allgemeinen Formel (II) 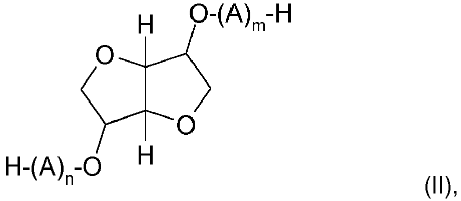 worin
- A: unabhängig voneinander Ethylenoxy-, Propylenoxy-, Butylenoxy-Einheiten und
- m + n: im Mittel Zahl von 0 bis 50
bedeuten.

Vorzugsweise gilt n = m. Die für m + n angegeben Zahlen sind in der Regel Mittelwerte für den Alkoxylierungsgrad.

In der allgemeinen Formel (II) kann A unabhängig voneinander Ethylenoxy-, Propylenoxy- oder Butylenoxy-Einheiten bedeuten.

Ethylenoxy-Einheiten entsprechen der Formel -CH₂-CH₂-O- und werden beispielsweise durch ringöffnende Addition von Ethylenoxid an den Alkohol durch dem Fachmann bekannte Verfahren, beispielsweise katalysiert durch Basen, Lewis-Basen oder Lewis-Säuren, erhalten.

Die Ethoxylierung kann mit gasförmigem Ethylenoxid in Gegenwart von basischen Katalysatoren bei einem Druck von im Allgemeinen 1 bis 10 bar und Temperaturen von 80 bis 200°C durchgeführt werden. Geeignete basische Katalysatoren sind beispielsweise NaOH, KOH, Natrium- oder Kaliummethylat oder Kalium-tert.-butanolat.

Propylenoxy-Einheiten entsprechen der Formel -C(CH₃)H-CH₂-O- oder -CH₂-C(CH₃)H-O- und werden beispielsweise durch ringöffnende Addition von Propylenoxid an den Alkohol durch dem Fachmann bekannte Verfahren, beispielsweise katalysiert durch Basen, Lewis-Basen oder Lewis-Säuren, erhalten.

Butylenoxy-Einheiten entsprechen der Formel -C(CH₃)H-CH₂-CH₂-O-, -CH₂-C(CH₃)H-CH₂-O- oder -CH₂-CH₂-C(CH₃)H-O- und werden beispielsweise durch ringöffnende Addition des entsprechenden Butylenoxids an den Alkohol durch dem Fachmann bekannte Verfahren, beispielsweise katalysiert durch Basen, Lewis-Basen oder Lewis-Säuren, erhalten.

In einer bevorzugten Ausführungsform der vorliegenden Erfindung bedeutet A in der allgemeinen Formel (II) Ethylenoxy-Einheiten.

Im Allgemeinen werden pro mol Isosorbid 0 bis 50 mol, bevorzugt 0 bis 25 mol, stärker bevorzugt 1 bis 10 mol, besonders bevorzugt 2 bis 4 mol Ethylenoxid umgesetzt.

In das erfindungsgemäße Verfahren wird bevorzugt eine Verbindung der allgemeinen Formel (II) eingesetzt, in der m und n gleichzeitig 0 sind.

In das erfindungsgemäße Verfahren wird weiter bevorzugt eine Verbindung der allgemeinen Formel (II) eingesetzt, in der m + n eine Zahl von 2 bis 4 bedeutet, wobei A in diesem Fall bevorzugt Ethylenoxy-Einheiten bedeutet.

Die erfindungsgemäß bevorzugt eingesetzte Verbindung der allgemeinen Formel (II) kann in verschiedenen stereoisomeren Formen vorliegen, wobei auch hier die allgemeinen und bevorzugten Bedeutungen für A, m und n den oben genannten entsprechen. Erfindungsgemäß können auch Mischungen von verschiedenen Isomeren eingesetzt werden.

Bevorzugt wird in das erfindungsgemäße Verfahren die Verbindung der allgemeinen Formel (IIa) eingesetzt: 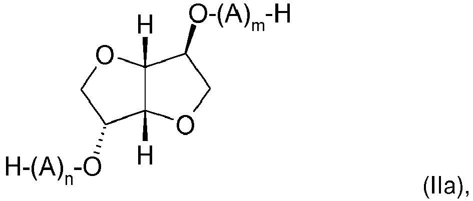 wobei A, m und n die oben genannten allgemeinen und bevorzugten Bedeutungen haben.

Alternativ zum Isosorbid der allgemeinen Formel (IIa) können auch Isoidid (exo-exo) oder Isomannid (endo-endo) eingesetzt werden.

Besonders bevorzugt werden als Verbindungen der allgemeinen Formel (IIa) die Verbindungen 1 D-Isosorbid bzw. (3R,3aR,6S,6aR)-Hexahydro-furo[3,2-b]furan-3,6-diol (IUPAC-Nomenklatur) und **2** ethoxyliertes D-Isosorbid bzw. (3R,3aR,6S,6aR)-Hexahydro-furo[3,2-b]furan-3,6-diol (IUPAC-Nomenklatur) eingesetzt:

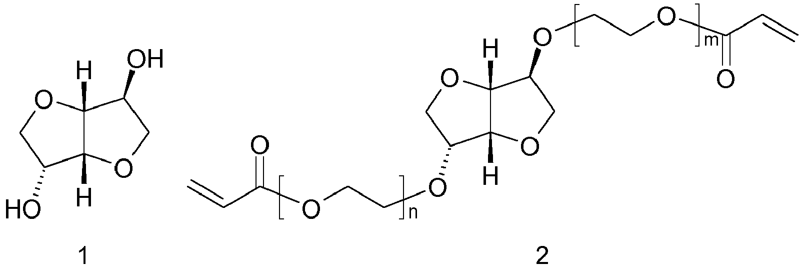

In der Verbindung **2** hat m + n einen Wert von 0 bis 50, vorzugsweise 0 bis 25, stärker bevorzugt 1 bis 10, besonders bevorzugt 2 bis 4. Vorzugsweise ist n = m.

Demnach sind besonders bevorzugt durch das erfindungsgemäße Verfahren herstellbare Verbindungen

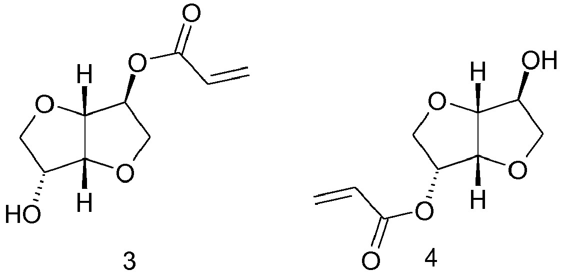

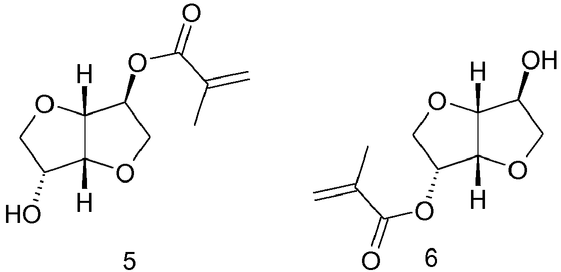

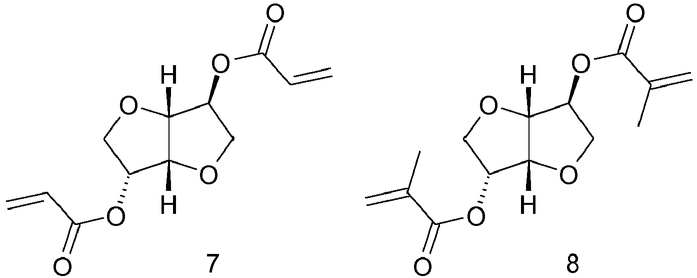

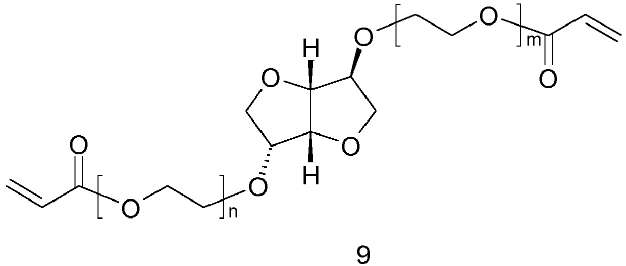

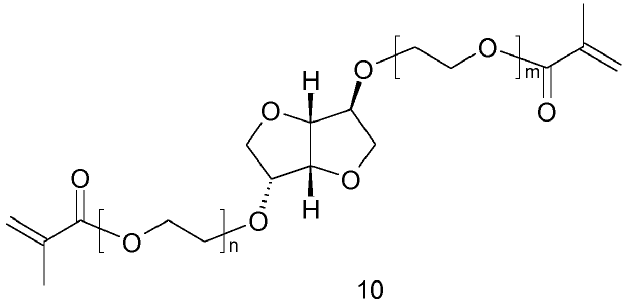

In den Verbindungen **9** und **10** hat m + n einen Wert von 0 bis 50, vorzugsweise 0 bis 25, stärker bevorzugt 1 bis 10, besonders bevorzugt 2 bis 4. Vorzugsweise ist n = m. Die Namen der Verbindungen 3 bis 10 lauten Isosorbid-mono-acrylsäureester (3 und 4), Isosorbid-mono-methacrylsäureester (5 und 6), Isosorbid-di-acrylsäureester (7), Isosorbid-di-methacrylsäureester (8), ethoxylierter Isosorbid-di-acrylsäureester (9) und ethoxylierter Isosorbid-di-methacrylsäureester (10).

Im erfindungsgemäßen Verfahren werden die (Meth)acrylsäure oder ein Derivat davon und das Isosorbid oder ein Derivat davon in einem molaren Verhältnis von 40 : 1 bis 5 : 1 eingesetzt.

In einer bevorzugten Ausführungsform des erfindungsgemäßen Verfahrens beträgt das molare Verhältnis von (Meth)acrylsäure oder einem Derivat davon und Isosorbid oder einem Derivat davon 30 : 1 bis 8 : 1, besonders bevorzugt 25 : 1 bis 8 : 1.

Das erfindungsgemäße Verfahren wird bei einer Reaktionstemperatur von 10 bis 100 C durchgeführt. In einer bevorzugten Ausführungsform beträgt die Reaktionstemperatur 20 bis 90°C, bevorzugt 30 bis 70°C, besonders bevorzugt 40 bis 60°C.

Das erfindungsgemäße Verfahren wird in Gegenwart wenigstens eines die Veresterungsreaktion katalysierenden Enzyms durchgeführt. Als Enzyme können erfindungsgemäß bevorzugt Hydrolasen [EC 3.x.x.x], insbesondere Esterasen [EC 3.1.x.x.] und Proteasen [EC 3.4.x.x], eingesetzt werden. Bevorzugt sind die Carboxylester-Hydrolasen [EC 3.1.1.x]. Besonders bevorzugt werden Lipasen als Hydrolasen eingesetzt. Insbesondere werden Lipasen aus Achromobacter sp., Aspergillus sp., Burholderia sp., Candida sp., Mucor sp., Penicillium sp., Pseudomonas sp., Rhizopus sp., Thermomyces sp. oder Schweinepankreas verwendet. Die Enzyme und ihre Funktionen werden beispielsweise in Römpp online, 2002 (https://roempp.thieme.de), "Hydrolasen", "Lipasen" und "Proteasen" beschrieben.

Besonders bevorzugt wird erfindungsgemäß eine Lipase aus Candida Antartica (Candida Antartica Lipase B) eingesetzt als Enzym eingesetzt.

Das wenigstens eine Enzym wird erfindungsgemäß in einer Menge von 0,1 bis 20 Gew.-%, bevorzugt 0,5 bis 10 Gew.-%, besonders bevorzugt 0,7 bis 5 Gew.-%, jeweils bezogen auf das in der Reaktionsmischung vorliegende Isosorbid, eingesetzt.

Das wenigstens eine Enzym kann erfindungsgemäß mobilisiert oder immobilisiert eingesetzt werden. Erfindungsgemäß bevorzugt werden immobilisierte, d.h. auf einem Träger vorliegende Enzyme eingesetzt. Geeignete Trägermaterialien sind dem Fachmann an sich bekannt, insbesondere werden organische, polymere Trägermaterialien eingesetzt, um das in dem erfindungsgemäßen Verfahren eingesetzte, wenigstens eine Enzym, zu immobilisieren.

Die vorliegende Erfindung betrifft daher bevorzugt das erfindungsgemäße Verfahren, wobei das wenigstens eine Enzym auf einem Trägermaterial vorliegt.

Das Enzym ist in dem erfindungsgemäßen Verfahren bevorzugt auf einem geeigneten Träger immobilisiert. Dabei existieren im Allgemeinen fünf klassische Methoden der Immobilisierung von Enzymen, nämlich die Adsorption, die kovalente Bindung, der Membraneinschluss, der Geleinschluss und die Quervernetzung. Dabei können unterschiedliche Trägermaterialien eingesetzt werden, wobei die chemischen Wechselwirkungen der Trägeroberfläche mit dem Enzym so angepasst sein müssen, dass keine unerwünschten Nebenwirkungen, wie z.B. Inaktivierung, entstehen. Als feste Träger eignen sich prinzipiell verschiedene anorganische und organische Materialien, letztere können natürlichen oder synthetischen Ursprungs sein. Anorganische Träger weisen meistens eine hohe Druckstabilität auf, während organische Träger eine gute chemische Stabilität zeigen. Als anorganische Träger werden überwiegend poröse Materialien auf der Basis von Silicium- oder Aluminiumoxiden bzw. Gemischen daraus eingesetzt. Natürliche organische Träger sind beispielsweise Polysaccharide wie z.B. Cellulose, Stärke, Dextran, Agarose und Chitin. Aber auch Proteine wie Kollagen, Gelatine und Albumin kommen zur Anwendung. Als synthetische organische Polymere dienen Poly(meth)acrylate, Polyacrylamide, Vinyl- und Allylpolymere, Polyester oder Polyamide.

Ein Beispiel für ein besonders bevorzugt eingesetztes Trägermaterial ist ein makroporöses, Divinylbenzol-vernetztes Polymer in sphärischer Perlenform auf Basis von Methacrylat. Dieses bevorzugt eingesetzte Trägermaterial weist eine Korngröße (D₈₀) von 0,3 bis 1,5 mm, bevorzugt 0,31 bis 1,2 mm, und eine effektive Größe von 0,3 bis 0,6 mm, bevorzugt 0,3 bis 0,5 mm, auf. Die Dichte des bevorzugt eingesetzten Trägermaterials beträgt beispielsweise 1,0 bis 1,5 g/ml, bevorzugt 1,02 bis 1,1 g/ml. Der Wassergehalt des bevorzugt eingesetzten Trägermaterials beträgt beispielsweise 40 bis 80 Gew.-%, bevorzugt 50 bis 70 Gew.-%.

Die BET-Oberfläche des erfindungsgemäß bevorzugt eingesetzten organischen Trägermaterials beträgt beispielsweise 100 bis 200 m²/g, bevorzugt 110 bis 150 m²/g.

Das Porenvolumen des erfindungsgemäß bevorzugt eingesetzten Trägermaterials beträgt beispielsweise 0,2 bis 1,0 cm³/g, bevorzugt 0,4 bis 0,8 cm³/g.

Der Porendurchmesser der in dem erfindungsgemäß bevorzugt eingesetzten Trägermaterial vorliegenden Poren beträgt beispielsweise 0,05 bis 0,5 nm, bevorzugt 0,1 bis 0,3 nm.

Besonders bevorzugt werden in dem erfindungsgemäßen Verfahren Enzyme eingesetzt, die bereits auf einem geeigneten Träger immobilisiert sind. Derartige immobilisierte Enzyme, bevorzugt Lipasen, sind unter dem Handelsnamen Novozym® 435 (Lipase aus Candida antartica B) von der Firma Novozymes erhältlich.

Gemäß der bevorzugten Ausführungsform, bei der das wenigstens eine Enzym auf einem Trägermaterial vorliegt, liegt es im Allgemeinen in einer Menge von 1 bis 20 Gew.-%, bevorzugt 2 bis 15 Gew.-%, besonders bevorzugt 5 bis 12 Gew.-%, jeweils bezogen auf die Summe aus Enzym und Trägermaterial, vor.

Die im erfindungsgemäßen Verfahren eingesetzten Enzyme, die die Veresterungsreaktion katalysieren, können auch in situ im Verfahren aus entsprechenden Organismen erhalten werden. Als Organismen kommen dazu alle natürlich vorkommenden oder gentechnisch veränderten Mikroorganismen, einzelligen Lebewesen oder Zellen in Betracht, die die Umesterung oder Veresterung mittels einer Hydrolase [EC 3.x.x.x], bevorzugt einer Esterase [EC 3.1.x.x.] oder Protease [EC 3.4.x.x], bevorzugt einer Carboxylesterhydrolase [EC 3.1.1.x] und insbesondere einer Lipase, katalysieren. Es sind alle dem Fachmann bekannten Organismen einsetzbar, die Hydrolasen beinhalten. Bevorzugt werden Organismen eingesetzt, die als Hydrolasen Lipasen umfassen. Insbesondere finden Achromobacter sp., Aspergillus sp., Burholderia sp., Candida sp., Mucor sp., Penicillium sp., Pseudomonas sp., Rhizopus sp., Thermomyces sp. und Zellen aus Schweinepankreas Verwendung. Dabei kann es sich um die unveränderten Organismen selbst oder um gentechnisch veränderte Organismen handeln, die die Enzyme ursprünglich nicht oder nur ungenügend stark exprimieren und erst nach Veränderung eine genügend hohe Enzym-Aktivität und Produktivität aufweisen. Ferner können die Organismen durch die gentechnische Veränderung an die Reaktionsbedingungen und/oder Kultivierungsbedingungen angepasst werden.

Das erfindungsgemäße Verfahren kann im Allgemeinen in Anwesenheit oder in Abwesenheit eines Lösungsmittels durchgeführt werden.

Geeignete Lösungsmittel sind insbesondere organische Lösungsmittel, weiter bevorzugt solche, die an der erfindungsgemäß stattfindenden Veresterungs- bzw. Umesterungsreaktion nicht selbst teilnehmen.

Die vorliegende Erfindung betrifft daher insbesondere das erfindungsgemäße Verfahren, wobei es in Gegenwart eines Lösungsmittels, ausgewählt aus der Gruppe bestehend aus sekundären bzw. tertiären Alkoholen, beispielsweise iso-Propanol oder tert.-Butanol, Ethern, beispielsweise Methyl-tert.-butylether, cyclischen Ethern, beispielsweise Tetrahydrofuran (TMF), Alkanen, beispielsweise Heptan, Cycloalkanen, beispielsweise Cyclohexan, halogenierten Lösungsmitteln, beispielsweise CH₂Cl₂, CHCl₃, aromatischen Lösungsmitteln, beispielsweise Toluol, und Mischungen davon durchgeführt wird.

In einer bevorzugten Ausführungsform wird das erfindungsgemäße Verfahren in Abwesenheit eines zusätzlichen Lösungsmittels durchgeführt. In dieser bevorzugten Ausführungsform wirkt die (Meth)acrylsäure oder ein Derivat davon, welche bevorzugt in einem Überschuss gegenüber Isosorbid eingesetzt wird, als Lösungsmittel.

Im Laufe der erfindungsgemäßen Veresterungs- bzw. Umesterungsreaktion werden als Reaktionsprodukte beispielsweise Wasser bei Einsatz entsprechender (Meth)acrylsäuren oder Alkohole, beispielsweise Methanol, Ethanol, Propanol, bei Einsatz der entsprechenden Ester, insbesondere (Meth)acrylsäureester, gebildet. Um die Reaktionsgeschwindigkeit durch Verlagerung des chemischen Gleichgewichts weiter zu erhöhen, ist es erfindungsgemäß bevorzugt, dass das während der Reaktion gebildete Wasser oder die entsprechenden Alkohole kontinuierlich entfernt werden.

Das kontinuierliche Entfernen des während der Reaktion gebildeten Wassers oder der entsprechenden Alkohole kann im Allgemeinen nach allen dem Fachmann bekannten Methoden erfolgen, beispielsweise Einsatz eines Trockenmittels, einer hydrophoben Membran, destillative Abtrennung und Kombinationen davon.

Erfindungsgemäß bevorzugt betrifft die vorliegende Erfindung daher das erfindungsgemäße Verfahren, wobei es in Gegenwart eines Trockenmittels durchgeführt wird. Erfindungsgemäß einzusetzende Trockenmittel sind dem Fachmann an sich bekannt und beispielsweise ausgewählt aus der Gruppe bestehend aus Molsieben, Calciumchlorid, Blaugel, Magnesiumsulfat und Mischungen davon.

In einer bevorzugten Ausführungsform des erfindungsgemäßen Verfahrens, insbesondere wenn dieses in einem größeren Maßstab, beispielsweise Pilot Plant oder industrieller Maßstab, durchgeführt wird, werden während der Reaktion Wasser, Alkohole, beispielsweise Methanol, Ethanol und/oder Propanol, oder Mischungen davon destillativ entfernt. Entsprechende Vorrichtungen sind dem Fachmann an sich bekannt, beispielsweise Destillationskolonnen.

In einer bevorzugten Ausführungsform des erfindungsgemäßen Verfahrens wird es in Gegenwart wenigstens eines Stabilisators durchgeführt. Weiter bevorzugt wird ein Stabilisator eingesetzt, um die Polymerisationsneigung der eingesetzten Edukte, d.h. der (Meth)acrylsäure oder einem Derivat davon, bzw. der während der Reaktion entstehenden Produkte, d.h. der Ester von Isosorbid oder eines Derivats davon mit (Meth)acrylsäure oder einem Derivat davon, zu unterbinden bzw. zu unterdrücken.

Geeignete Stabilisatoren bzw. Polymerisationsinhibitoren können beispielsweise N-Oxide (Nitroxyl- oder N-Oxyl-Radikale, also Verbindungen, die wenigstens eine >N-O-Gruppe aufweisen), wie z.B. 4-Hydroxy-2,2,6,6-tetramethylpiperidin-N-oxyl, 4-Oxo-2,2,6,6-tetramethylpiperidin-N-oxyl, 4-Acetoxy-2,2,6,6-tetramethyl-piperidin-N-oxyl, 2,2,6,6-Tetramethylpiperidin-N-oxyl, 4,4',4"-Tris(2,2,6,6-tetramethyl-piperidin-N-oxyl)-phosphit oder 3-Oxo-2,2,5,5-tetramethyl-pyrrolidin-N-oxyl; ein- oder mehrwertige Phenole, die ggf. eine oder mehrere Alkylgruppen aufweisen, wie z.B. Alkylphenole, beispielsweise o-, m- oder p-Kresol (Methylphenol), 2-tert.-Butylphenol, 4-tert.-Butylphenol, 2,4-Di-tert.-butylphenol, 2-Methyl-4-tert.-butylphenol, 2-tert.-Butyl-4-methylphenol, 2,6-tert.-Butyl-4-methylphenol, 4-tert.-Butyl-2,6-dimethylphenol oder 6-tert.-Butyl-2,4-dimethylphenol; Chinone, wie z.B. Hydrochinon, Hydrochinonmonomethylether, 2-Methylhydrochinon oder 2,5-Di-tert.-Butylhydrochinon; Hydroxyphenole, wie beispielsweise Brenzcatechin (1,2-Dihydroxybenzol) oder Benzochinon; Aminophenole, wie z.B. p-Aminophenol; Nitrosophenole, wie z.B. p-Nitrosophenol; Alkoxyphenole, wie beispielsweise 2-Methoxyphenol (Guajacol, Brenzcatechinmonomethylether), 2-Ethoxyphenol, 2-Isopropoxyphenol, 4-Methoxyphenol (Hydrochinonmonomethylether), Mono- oder Di-tert.-Butyl-4-methoxyphenol; Tocopherole, wie z.B. α-Tocopherol sowie 2,3-Dihydro-2,2-dimethyl-7-hydroxybenzofuran (2,2-Dimethyl-7-hydroxycumaran), aromatische Amine, wie z.B. N,N-Diphenylamin oder N-Nitroso-diphenylamin; Phenylendiamine, wie z.B. N,N'-Dialkyl-p-phenylendiamin, wobei die Alkylreste gleich oder verschieden sein können und jeweils unabhängig voneinander aus 1 bis 4 Kohlenstoffatomen bestehen und geradkettig oder verzweigt sein können, wie z.B. N,N'-Dimethyl-p-phenylendiamin oder N,N'-Diethyl-p-phenylendiamin, Hydroxylamine, wie z.B. N,N-Diethylhydroxylamin, Imine, wie z.B. Methylethylimin oder Methylen violett, Sulfonamide, wie z.B. N-Methyl-4-toluolsulfonamid oder N-tert.-Butyl-4-toluolsulfonamid, Oxime, wie Aldoxime, Ketoxime oder Amidoxime, wie z.B. Diethylketoxim, Methylethylketoxim oder Salicyladoxim, phosphorhaltige Verbindungen, wie z.B. Triphenylphosphin, Triphenylphosphit, Triethylphosphit, Hypophosphorige Säure oder Alkylester der Phosphorigen Säuren; schwefelhaltige Verbindungen wie z.B. Diphenylsulfid oder Phenothiazin; Metallsalze, wie Kupfer- oder Mangan-, Cer-, Nickel-, Chromsalze, beispielsweise -chloride, -sulfate, -salicylate, -tosylate, -acrylate oder -acetate, wie z.B. Kupferacetat, Kupfer(II)chlorid, Kupfersalicylat, Cer(III)acetat oder Cer(III)ethylhexanoat, oder Gemische davon sein.

Besonders geeignete Stabilisatoren für das erfindungsgemäße Verfahren sind Hydrochinon, Hydrochinonmonomethylether (MEHQ), Phenothiazin, 4-Hydroxy-2,2,6,6-tetra-methylpiperidin-N-oxyl, 4-Oxo-2,2,6,6-tetramethylpiperidin-N-oxyl, 2-tert.-Butylphenol, 4-tert.-Butylphenol, 2,4-Di-tert.-butylphenol, 2-tert.-Butyl-4-methylphenol, 6-tert.-Butyl-2,4-dimethyl-phenol, 2,6-Di-tert.-butyl-4-methylphenol, 2-Methyl-4-tert.-butylphenol, hypophosphorige Säure, Kupferacetat, Kupfer(II)chlorid, Kupfersalicylat und Cer(III)acetat. Insbesondere bevorzugte Stabilisatoren sind Hydrochinonmonomethylether (MEHQ) und/oder Phenothiazin (PTZ).

Im Allgemeinen werden, jeweils bezogen auf die ungesättigten Monomere, je Einzelsubstanz 1 bis 10 000 ppm, bevorzugt 10 bis 5 000 ppm, besonders bevorzugt 30 bis 2 500 ppm und insbesondere 50 bis 1 500 ppm, eines geeigneten Polymerisationsinhibitors eingesetzt.

Das erfindungsgemäße Verfahren kann im Allgemeinen bei einem Druck von 0,01 bis 1,5 bar (a) durchgeführt werden. Bevorzugt wird das Verfahren bei einem Druck durchgeführt, der unterhalb Atmosphärendruck liegt, d.h. bei einem Druck von 0,01 bis ca. 0,9 bar (a). In der bevorzugten Ausführungsform, dass während des erfindungsgemäßen Verfahrens das entstehende Wasser bzw. niedermolekulare Alkohole kontinuierlich destillativ entfernt werden, wird das Verfahren insbesondere bei einem Druck von 0,01 bis 0,5 bar (a) durchgeführt, um den Siedepunkt der entstehenden niedermolekularen Verbindungen weiter herabzusenken. Das erfindungsgemäße Verfahren wird in einer bevorzugten Ausführungsform bei Atmosphärendruck durchgeführt.

Nach erfolgter Reaktion kann die erhaltene Reaktionsmischung im Allgemeinen nach allen dem Fachmann bekannten Methoden aufgearbeitet werden, beispielsweise Filtration, destillatives Entfernen eines gegebenenfalls vorhandenen Lösungsmittels etc.

In einer bevorzugten Ausführungsform des erfindungsgemäßen Verfahrens schließt sich an die Umsetzung von Isosorbid oder eines Derivats davon mit (Meth)acrylsäure oder einem Derivat davon eine Extraktion des erhaltenen Reaktionsprodukt mit wenigstens einem organischen Lösungsmittel an, um die bei der Umsetzung entstehenden Mono- und Diester voneinander zu trennen.

Nach der erfindungsgemäßen Umsetzung von Isosorbid oder einem Derivat davon mit (Meth)acrylsäure oder einem Derivat davon wird im Allgemeinen eine Mischung enthaltend den entsprechenden Monoester, d.h. eine der beiden Hydroxyfunktionen des Isosorbids oder eines Derivats davon sind verestert, und den entsprechenden Diester, d.h. beide Hydroxyfunktionen des Isosorbids oder eines Derivats davon sind verestert, erhalten.

Es wurde überraschenderweise erfindungsgemäß gefunden, dass diese Mischung in die entsprechenden Mono- und Diester getrennt werden kann, indem das erhaltene Reaktionsprodukt mit wenigstens einem Lösungsmittel extrahiert wird. In einer bevorzugten Ausführungsform wird das erhaltene Reaktionsprodukt mit wenigstens zwei unterschiedlichen Lösungsmittels extrahiert.

Bevorzugt wird die Reaktionsmischung nach Beendigung der Reaktion aufgearbeitet, beispielsweise durch Filtration, destillatives Entfernen eines gegebenenfalls vorhandenen Lösungsmittels etc. Anschließend wird das so erhaltene Produkt bevorzugt in Wasser oder einer wässrigen Lösung, bevorzugt in Wasser, aufgenommen.

Bevorzugt wird mit dem erhaltenen Produkt, welches in Wasser oder eine wässrigen Lösung aufgenommen worden ist, die erfindungsgemäß bevorzugte Extraktion durchgeführt.

Bevorzugt wird die wässrige Phase enthaltend das Produkt und Wasser oder eine wässrige Lösung in einem ersten Extraktionsschritt einmal oder mehrmals mit einem unpolaren, organischen Lösungsmittel, beispielsweise ausgewählt aus der Gruppe bestehend aus Alkanen, insbesondere n-Hexan, Cyclohexan, n-Heptan, aromatischen Lösungsmitteln, beispielsweise Toluol, Xylol und Mischungen davon, extrahiert. Die Extraktion wird einmal oder mehrmals, bevorzugt zweimal, dreimal, viermal, durchgeführt.

In diesem ersten Extraktionsschritt wird bevorzugt der entsprechende Diester aus der wässrigen Phase extrahiert.

Die nach dem ersten Extraktionsschritt verbliebene wässrige Phase wird bevorzugt mit einem weiteren Lösungsmittel extrahiert. In diesem zweiten Extraktionsschritt wird bevorzugt ein polares, organisches Lösungsmittel, beispielsweise ausgewählt aus der Gruppe bestehend aus Estern, insbesondere Essigsäureethylester (Essigester), oder n-Butylacetat, und Mischungen davon, eingesetzt. Die Extraktion wird einmal oder mehrmals, bevorzugt zweimal, dreimal, viermal, durchgeführt.

In diesem zweiten Extraktionsschritt wird bevorzugt der entsprechende Monoester aus der wässrigen Phase extrahiert.

Die Extraktionen an sich können nach dem Fachmann bekannten Methoden und in bekannten Vorrichtungen erfolgen.

Die Abtrennung der gewünschten Produkte aus den einzelnen in den Extraktionsschritten erhaltenen Fraktionen kann nach dem Fachmann bekannten Verfahren erfolgen, beispielsweise durch destillatives Abtrennen des Extraktionsmittels.

Die vorliegende Erfindung betrifft des Weiteren auch einen entsprechenden Ester, herstellbar, bevorzugt hergestellt, nach dem erfindungsgemäßen Verfahren.

Das bezüglich der Edukte (Meth)acrylsäure oder einem Derivat davon und Isosorbid oder einem Derivat davon und der erhaltenen Produkte Gesagte gilt hier entsprechend.

Insbesondere betrifft die vorliegende Erfindung die folgenden Verbindungen:

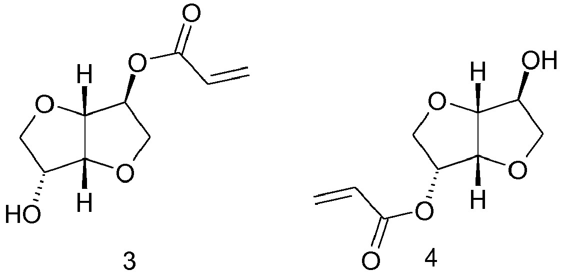

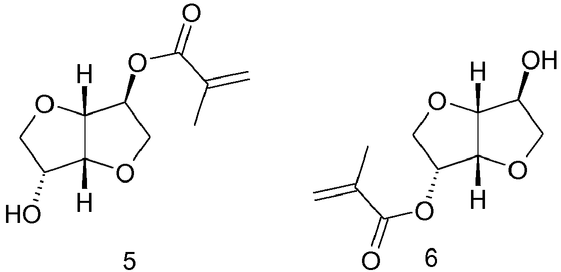

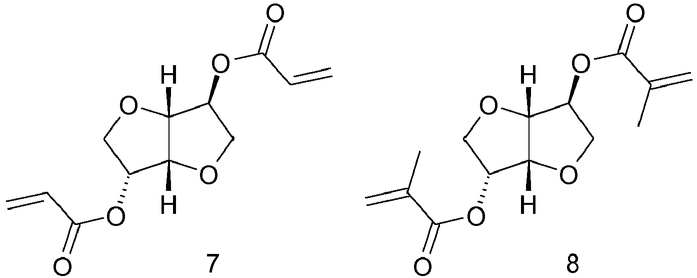

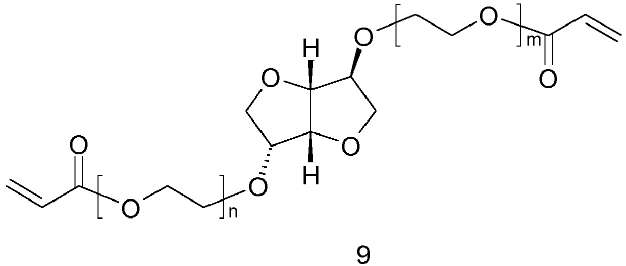

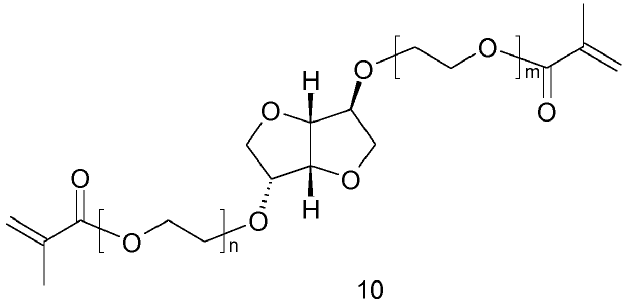

In den Verbindungen 9 und 10 hat m + n einen Wert von 0 bis 50, vorzugsweise 0 bis 25, stärker bevorzugt 1 bis 10, besonders bevorzugt 2 bis 4. Vorzugsweise ist n = m.

Die erfindungsgemäß hergestellten Produkte zeichnen sich gegenüber den entsprechenden Produkten, hergestellt nach Verfahren des Standes der Technik, durch explizite Difunktionalisierung aus.

Die vorliegende Erfindung betrifft daher auch die Verwendung eines erfindungsgemäßen Esters in Kontaktlinsen oder als Vernetzer oder Haftverbesserer für Dispersionen, die vorzugsweise als Klebstoffe, Anstrichmittel oder Textil-, Leder- oder Papierhilfsmittel und in härtbaren Beschichtungen eingesetzt werden.

### Beispiele

### Beispiel 1:

In einer 500 ml Schottflasche wurden Isosorbid (20 g, 0,14 mol), Acrylsäuremethylester (234 g, 2,72 mol), Molekularsieb 5A (87 g), MeHQ (0,008 g, 400 ppm) und Novozym 435 (1,5 g, 7,5 Gew.-%, geträgertes Enzym aus Candida Antarctica auf Divinylbenzol-vernetztem Polymer in sphärischer Perlenform auf Basis von Methacrylat, 10 Gew.-% Enzym, bezogen auf das Trägermaterial) eingewogen. Der Ansatz wurde bei 60°C im Wasserbadschüttler geschüttelt. Der Umsatz wurde per Gaschromatographie kontrolliert. Die Ergebnisse sind in Tabelle 1 dargestellt:

**Tabelle 1:**

| | 1 h [Fl.-%] | 2 h [Fl.-%] | 4 h [Fl.-%] | 6,5 h [Fl.-%] | 23 h [Fl.-%] | 29 h [Fl.-%] | 2 Tage [Fl.-%] | 3 Tage [Fl.-%] | 7 Tage [Fl.-%] |
|---|---|---|---|---|---|---|---|---|---|
| Isosorbid (Edukt) | 100 | 92,7 | 86,3 | 77,3 | 39,6 | 33,0 | 11,9 | 12,1 | 9,0 |
| Isosorbidmonoacrylat (Produkt I) | 0 | 7,3 | 13,7 | 22,7 | 47,5 | 49,3 | 66,9 | 58,0 | 54,8 |
| Isosorbiddiacrylat (Produkt II) | 0 | 0 | 0 | 0 | 6,8 | 10,3 | 21,2 | 24,4 | 29,0 |

| | | | | | | | | | |
|---|---|---|---|---|---|---|---|---|---|
| Fl.-%: Flächenprozent | | | | | | | | | |

### Beispiel 2:

In einer 500 ml Schottflasche wurde Isosorbid (20 g, 0,14 mol), Acrylsäure-methyl-ester (117,8 g, 1,37 mol), Molekularsieb 5A (87 g), MeHQ (0,008 g, 400 ppm) und Novozym 435 (1,5 g, 7,5 Gew.-%, geträgertes Enzym aus Candida Antarctica auf Divinylbenzol-vernetztem Polymer in sphärischer Perlenform auf Basis von Methacrylat, 10 Gew.-% Enzym, bezogen auf das Trägermaterial) eingewogen. Der Ansatz wurde bei 60°C im Wasserbadschüttler geschüttelt. Den Umsatz wurde per GC (siehe Beispiel 1) kontrolliert. Die Ergebnisse sind in Tabelle 2 dargestellt.

**Tabelle 2:**

| | 5 Tage [Fl.-%] |
|---|---|
| Isosorbid (Edukt) | 0 |
| Isosorbidmonoacrylat (Produkt I) | 66,9 |
| Isosorbiddiacrylat (Produkt II) | 21,2 |

### Beispiel 2.1:

Die Reaktionsmischung aus Beispiel 2 wurde über Kieselgel abgesaugt, mit Aceton nachgewaschen, Phenothiazin in einer Menge von 50 bis 100 ppm, bezogen auf die Mischung, zugegeben und am Rotationsverdampfer bei höchstens 40°C und unter Luftdurchleiten eingeengt. 13,25 g Produkt wurde gewonnen. Es wurden 100 ml Wasser zugegeben, und die Mischung wurde viermal mit je 100 ml n-Hexan extrahiert. Die so erhaltene wässrige Phase wurde anschließend viermal mit je 100 ml Essigsäureethylester (EE) extrahiert. Die einzelnen organischen Phasen wurden per GC (siehe Beispiel 1) analysiert. Die Ergebnisse sind in Tabelle 3 dargestellt:

**Tabelle 3:**

| | Isosorbid [Fl.-%] | Isosorbidmonoacrylat [Fl.-%] | Isosorbiddiacrylat [Fl-.%] |
|---|---|---|---|
| n-Hexan extr. 1 | 0 | 0 | 100 |
| n-Hexan extr. 2 | 0 | 0 | 100 |
| n-Hexan extr. 3 | 0 | 0 | 100 |
| n-Hexan extr. 4 | 0 | 0 | 100 |
| EE extr.1 | 0 | 65,73 | 29,702 |
| EE extr. 2 | 0 | 95,1 | 2,3 |
| EE extr. 3 | 0 | 100 | 0 |
| EE extr. 4 | 0 | 100 | 0 |

Durch Beispiel 2.1 kann gezeigt werden, dass sich die in der Reaktionsmischung vorliegenden Mono- und Diester extraktiv trennen lassen.

### Beispiel 3:

In einer 500 ml Schottflasche wurden Isosorbid (20 g, 0,14mol), Methylmethacrylat (272 g, 2,72 mol), Molekularsieb 5A (87 g), MeHQ (0,008 g, 400 ppm) und Novozym 435 (1,5 g, 7,5 Gew.-%, geträgertes Enzym aus Candida Antarctica auf Divinylbenzol-vernetztem Polymer in sphärischer Perlenform auf Basis von Methacrylat, 10 Gew.-% Enzym, bezogen auf das Trägermaterial) eingewogen. Der Ansatz wurde bei 60°C im Wasserbadschüttler geschüttelt. Den Umsatz wurde per GC (siehe Beispiel 1) kontrolliert. Die Ergebnisse sind in Tabelle 4 dargestellt:

**Tabelle 4:**

| | 1 h [Fl.-%] | 2 h [Fl.-%] | 4 h [Fl.-%] | 6,5 h [Fl.-%] | 24 h [Fl.-%] | 2 Tage [Fl.-%] | 3 Tage [Fl.-%] | 7 Tage [Fl.-%] |
|---|---|---|---|---|---|---|---|---|
| Isosorbid | 100 | 100 | 96,9 | 93,8 | 71,5 | 47,4 | 20,6 | 0 |
| Isosorbidmonomethacrylat | 0 | 0 | 3,1 | 6,2 | 28,5 | 52,6 | 69,8 | 46,1 |
| Isosorbiddimethacrylat | 0 | 0 | 0 | 0 | 0 | 0 | 9,6 | 53,9 |

Der Ansatz wird über Kieselgel abgesaugt, mit Aceton nachgewaschen, Phenothiazin in einer Menge von 50 bis 100 ppm, bezogen auf die Mischung, zugegeben und am Rotationsverdampfer bei < 40°C und unter Luftdurchleiten eingeengt. 29,17 g Produkt wurde gewonnen.

Das Produkt wird in 100 ml Wasser gelöst und viermal mit je 100 ml Toluol, danach viermal mit je 100 ml Essigester extrahiert. Die Ergebnisse sind in Tabelle 5 dargestellt:

**Tabelle 5:**

| | Tol 1 [Fl.-%] | Tol 2 [Fl.-%] | Tol 3 [Fl.-%] | Tol 4 [Fl.-%] | EE 1 [Fl.-%] | EE 2 [Fl.-%] | EE 3 [Fl.-%] | EE 4 [Fl.-%] |
|---|---|---|---|---|---|---|---|---|
| Isosorbid | 0,3 | 0 | 0 | 0 | 0 | 0 | 0 | 0 |
| Isosorbidmonomethacrylat | 19,3 | 56,1 | 88,3 | 100 | 100 | 100 | 100 | 100 |
| Isosorbiddimethacrylat | 80,4 | 43,9 | 11,7 | 0 | 0 | 0 | 0 | 0 |

### Beispiel 4:

In einer 50 ml Schottflasche wurden ethoxyliertes Isosorbid (5 g, 0,018 mol), entsprechend 3 E0-Einheiten, Methylacrylat (15,48 g, 0,18 mol), Molekularsieb 5A (11,52 g), MeHQ (0,002 g, 400 ppm) und Novozym 435 (0,375g, 7,5 Gew-%, geträgertes Enzym aus Candida Antarctica auf Divinylbenzol-vernetztem Polymer in sphärischer Perlenform auf Basis von Methacrylat, 10 Gew.-% Enzym, bezogen auf das Trägermaterial) eingewogen. Der Ansatz wurde bei 60°C im Wasserbadschüttler geschüttelt. Den Umsatz wurde per GC (siehe Beispiel 1) kontrolliert. Die Ergebnisse sind in Tabelle 6 dargestellt:

**Tabelle 6:**

| | 5h Fl.-% | 24 h Fl.-% | 48 h Fl.-% |
|---|---|---|---|
| Produkt 1 | 25,48 | 26,36 | 23,81 |
| Produkt 2 | 27,64 | 28,16 | 28,17 |
| Produkt 3 | 6,83+6,45 | 6,47+6,02 | 8,07+7,86 |

| | | | |
|---|---|---|---|
| Produkt 1: Diacrylat+2EO Produkt 2: Diacrylat+3EO Produkt 3: Diacrylat+4EO | | | |

Die Gesamtmenge an Produkt, d.h. Produkt 1 + Produkt 2 + Produkt 3, ändert sich nach 5 h nicht mehr signifikant.

### Beispiel 5:

In einer 50 ml Schottflasche wurden ethoxyliertes Isosorbid (5 g, 0,018 mol), Methylmethacrylat (18,02 g, 0,18 mol), Molekularsieb 5A (11,52 g), MeHQ (0,002 g, 400 ppm) und Novozym 435 (0,375 g, 7,5 Gew.-%, geträgertes Enzym aus Candida Antarctica auf Divinylbenzol-vernetztem Polymer in sphärischer Perlenform auf Basis von Methacrylat, 10 Gew.-% Enzym, bezogen auf das Trägermaterial) eingewogen. Der Ansatz wurde bei 60°C im Wasserbadschüttler geschüttelt. Den Umsatz wurde per GC (siehe Beispiel 1) kontrolliert:

| | 5h [Fl.-% | 24 h [Fl.-%] | 48 h [Fl.-%] |
|---|---|---|---|
| Produkt 1 | 21,39 | 20,91 | 21,1 |
| Produkt 2 | 27,93 | 26,29 | 26,64 |
| Produkt 3 | 9,19+8,11 | 8,32+7,30 | 8,34+7,54 |

| | | | |
|---|---|---|---|
| Produkt 1: Diacrylat+2EO Produkt 2: Diacrylat+3EO Produkt 3: Diacrylat+4EO | | | |

Die Gesamtmenge an Produkt, d.h. Produkt 1 + Produkt 2 + Produkt 3, ändert sich nach 5 h nicht mehr signifikant.

## Patentansprüche

1. Verfahren zur Herstellung von Estern von Isosorbid oder eines Derivats von Isosorbid durch Umsetzung von Isosorbid oder eines Derivats davon mit (Meth)acrylsäure oder einem Derivat davon in Gegenwart wenigstens eines die Veresterungsreaktion katalysierenden Enzyms bei einer Reaktionstemperatur von 10 bis 150°C.

2. Verfahren nach Anspruch 1, **dadurch gekennzeichnet, dass** das Isosorbid oder das Derivat davon der allgemeinen Formel (II) 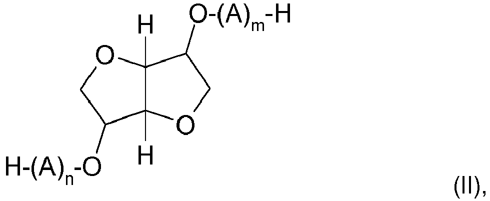 entspricht, worin
A unabhängig voneinander Ethylenoxy-, Propylenoxy-, Butylenoxy-Einheiten und
m + n Zahl von 0 bis 50
bedeuten.

3. Verfahren nach Anspruch 1 oder 2, **dadurch gekennzeichnet, dass** der entsprechende Mono-, der entsprechende Diester von Isosorbid oder eines Derivats davon oder eine Mischung davon gebildet wird.

4. Verfahren nach einem der Ansprüche 1 bis 3, **dadurch gekennzeichnet, dass** eine Lipase aus Candida Antartica als Enzym eingesetzt wird.

5. Verfahren nach einem der Ansprüche 1 bis 4, **dadurch gekennzeichnet, dass** das wenigstens eine Enzym auf einem Trägermaterial vorliegt.

6. Verfahren nach einem der Ansprüche 1 bis 5, **dadurch gekennzeichnet, dass** als Derivat der (Meth)acrylsäure ein Ester, insbesondere ein Methyl-, Ethyl- oder Propylester, eingesetzt wird.

7. Verfahren nach einem der Ansprüche 1 bis 6, **dadurch gekennzeichnet, dass** es in Gegenwart eines Lösungsmittels ausgewählt aus der Gruppe bestehend aus sekundären bzw. tertiären Alkoholen, Ethern, cyclischen Ethern, Alkanen, Cycloalkanen, halogenierten Lösungsmitteln, aromatischen Lösungsmitteln und Mischungen davon durchgeführt wird.

8. Verfahren nach einem der Ansprüche 1 bis 7, **dadurch gekennzeichnet, dass** das Verfahren in Gegenwart eines Trockenmittels durchgeführt wird.

9. Verfahren nach einem der Ansprüche 1 bis 8, **dadurch gekennzeichnet, dass** es in Gegenwart wenigstens eines Stabilisators durchgeführt wird.

10. Verfahren nach einem der Ansprüche 1 bis 9, **dadurch gekennzeichnet, dass** während der Reaktion Wasser, Alkohole oder Mischungen davon destillativ entfernt werden.

11. Verfahren nach einem der Ansprüche 1 bis 10, **dadurch gekennzeichnet, dass** sich an die Umsetzung von Isosorbid oder eines Derivats davon mit (Meth)acrylsäure oder einem Derivat davon eine Extraktion des erhaltenen Reaktionsprodukt mit wenigstens einem organischen Lösungsmittel anschließt, um die bei der Umsetzung entstehenden Mono- und Diester voneinander zu trennen.

12. Ester herstellbar nach dem Verfahren gemäß einem der Ansprüche 1 bis 11.

13. Verwendung eines Esters gemäß Anspruch 12 in Kontaktlinsen oder als Vernetzer oder Haftverbesserer für Dispersionen die vorzugsweise als Klebstoffe, Anstrichmittel oder Textil-, Leder- oder Papierhilfsmittel sowie in härtbaren Beschichtungen eingesetzt werden.
